# EUROPEAN PATENT APPLICATION

(11) **EP 1 970 051 A1**
(43) Date of publication of application: **17.09.2008**
(21) Application number: 07005271.7
(22) Date of filing: 14.03.2007
(51) Int. Cl.: A61K 9/107, A61K 31/205, A61K 31/575, A61K 31/765, A61P 3/00

(54) **Use of an aqueous micro-emulsion for the preparation of a formulation for the treatment of adipose diseases**

(71) Applicant: Merz Pharma GmbH & Co.KGaA, 60318 Frankfurt (DE)
(72) Inventor: Mentrup, Edgar, Dr., 60437 Frankfurt am Main (DE); Pooth, Rainer, Dr., 65812 Bad Soden/Taunus (DE); Wimmer, Thomas, Dr., 60489 Frankfurt am Main (DE); Drewes, Sigrid, Dr., 69121 Heidelberg (DE)
(74) Representative: Michalski Hüttermann & Partner Patentanwälte

(57) **Abstract**

Use of a micro-emulsion comprising at least one surfactant having a HLB value between 5 and 15, at least one lipophilic substance, and water for the preparation of a formulation for the treatment of adipose tissue disease and/or condition with improved bioavailability and good release behaviour of active substances.

## Description

The present invention relates to the use of an aqueous micro-emulsion comprising at least one surfactant having a HLB value between 5 and 15, at least one lipophilic substance and water for the production of a drug for the treatment of adipose diseases and/or conditions.

In general, micro-emulsions comprising of surfactants, lipophilic substances and water are well known in the field of pharmaceutical formulations to act as stable carrier for drugs that show poor water solubility. In such known micro-emulsions there is always a special drug that dictates the choice of the other components. They should be tailored to the desired pathway of the part of the body where the drug is most effective and they should not to interact with the drug.
Thus, any effect of the components of the micro-emulsion formulation other than the carrier function is undesired.

On the other hand, aqueous systems of phospholipids and bile acid or its derivatives are well known for the preparation of cosmetic and pharmaceutical formulations.
EP 0 615 746 A1 and WO 2005/112942 A1 describe such formulations that can carry a pharmaceutically active substance or that can be used without such an active drug. In the latter case, it is described that such liposomes can be used for the treatment of atherosclerosis, elevated blood lipids, and hepatopathy of any kind.
The described systems show a distinct liposome structure, i.e. a double membrane of lipids that encapsulates an aqueous phase.
In recent literature it is further described that such liposome systems can reduce fatty tissue when locally injected subcutaneously (Patricia Guedes Rittes, The use of phosphatidylcholine for correction of lower lid bulging due to prominent fat pads, Dermatol Surg 2001, 27, 391-392).
Further, a special liposome system for the prophylaxis and treatment of fatty embolism is known that comprises phospholipids, bile acid, DL-alpha-Tocopherole, ethanol and water (Lipostabil ® N i.V.).

However, the known aqueous liposome systems of phospholipids and bile acid or its derivatives for the treatment of reducing fatty tissue have the distinct disadvantage that their distribution inside the tissue is poor and thus the effect is fairly locally constricted to the immediate point of injection. Accordingly, up to date it is necessary for the treatment of a wider area of tissue to apply a high number of injections close to each other.

Therefore, it is an object of the present invention to provide a formulation for the treatment of adipose diseases and/or conditions that show a good biocompatibility and an enhanced bioavailability such that wider areas of fatty tissue can be affected.

Surprisingly, it was found that the use of a micro-emulsion comprising of at least one surfactant having an HLB value between 5 and 15, at least one lipophilic substance and water for the preparation of a formulation for the treatment of adipose diseases and/or conditions meets the object of the present invention.

The inventive use of the micro-emulsions of the present invention shows a better bioavailability and a better distribution in the fatty tissue. Thus, it allows for fewer injections when a wider area of tissue is to be treated and in general, a better effect of lipolysis.
It is thought that the advantageous effects are derived due to the differences in the structure of the ternary micro-emulsion system with its distinct properties like thermo dynamical stability and distribution as well as size of different phase droplets compared to the liposome systems of the state of the art. However, the transport mechanisms involved are not well understood so that scientifically sound evidence for the mechanism is yet to be found.

Micro-emulsions are clear, isotropic liquid mixtures of water, oil and surfactant. The water phase may contain salts and/or other ingredients. It is possible to prepare micro-emulsions from a large amount of components. In contrast to ordinary emulsions micro-emulsions form upon simple mixing of the components and do not require high shear conditions. In ternary systems such as micro-emulsions where two immiscible phases (water and "oil") are present next to the surfactant phase, the surfactant molecules form a monolayer at the interface between oil and water. The hydrophobic part of the surfactant molecules are dissolved in the oil phase and the hydrophilic part of the surfactant molecules are in the aqueous phase. Micro-emulsions are thermodynamically stabilized by the surfactant in a special way because they are not simply a dispersion of droplets of oil in water or vice-versa but a more complex mixture of solute, solution, reversed and normal micelles, and micro-emulsion droplets.

The droplet size of the micro-emulsions of the present invention is preferably between 10 nm and 200 nm, more preferably between 30 nm and 100 nm.

The micro-emulsion according to the present invention is preferably transparent or light opaque. Micro-emulsions are single phased in a given range of pressure, temperature, and composition. In contrast to emulsions they are thermodynamically stable systems due to their small particle sizes and they have the advantage that they build spontaneously and are stable even if stored for a long time.
Subject to the type of surfactant used micro-emulsions are distinguished into ionic and non-ionic micro-emulsions.

In the term "HLB value" HLB stands for hydrophile-lipophile balance. Surfactants with a low HLB are more lipophilic and thus tend to make a water in oil emulsion while those with a high HLB are more hydrophilic and tend to make an oil in water emulsion. The HLB value of each surfactant is determined by an analysis of the characteristics of the surfactant. A list of HLB values for various surfactants is available in many references such as the Handbook of Pharmaceutical Excipients, 3rd Edition. The HLB value can be used to predict the surfactant properties of a molecule, typically a value from 3 to 6 indicates a W/O emulsifier, a value from 7 to 9 indicates a wetting agent, a value from 8 to 12 indicates an O/W emulsifier, a value from 12 to 15 is typical of detergents, and a value of 15 to 20 indicates a solubiliser or hydrotrope.

Preferably, the surfactant of the present invention can be selected from the group comprising 3-sn-phosphatidylcholine, soy (phospholipone 90), reduced soy (phospholipon 90H), 3-(3sn)-phosphatidylgycerol soy (phospholipon G), dimyristoylphosphatidylglycerole, lyso-phophatidylcholine, dipalmitoylphosphatidylglycerole, and/or their physiologically acceptable salts, desoxycholic acid, cholic acid, lithocholic acid, chendodesoxycholic acid, hyodesoxycholic acid, trihydroxycoprostanic acid, ursodesoxycholic acid, taurocholic acid, or glycocholic acid and/or their physiologically acceptable salts, as well as ethers of ethoxylated alcohols and alkyl-alcohols (C6-C16), alkyl-ester with C8-C20 with ethoxylated alcohols, ester of saturated and unsatured acids with C8-C20 with sugars, alkylethersulfates like polyether of caster oil and ethylene oxide (cremephor EL), polyoxyethylene fatty alcohol ether, polysorbic monoester, poloxamer, poloxamine.

More preferably, the surfactant has an HLB value of between 7 and 13, more preferably between 9 and 11.

The lipophilic substance of the inventive micro-emulsion system can be selected from the group comprising natural oils (e.g. soy bean oil), ester of middle-chain alkyl acids with glycols, octyl-odecanol, silicon oils, paraffins, fatty acids and/or their esters, riboflavine, and/or L-camitine.

Due to the provision of a lipophilic substance in the micro-emulsion an even better distribution of the active substances can take place. Thus, wider areas of tissue can be affected by a single injection.

In a preferred embodiment of the present invention the system further comprises a co-surfactant.
In general, the co-surfactant is less lipophilic than the surfactant with an HLB value of 9 to 17.

Like that, a further stabilisation of the inventive micro-emulsion system can be achieved resulting in a longer shelf-life.

The co-surfactant can in particular be chosen from the group of 3-sn-phosphatidylcholine, soy (phospholipone 90), reduced soy (phospholipon 90H), 3-(3sn)-phosphatidylgycerol soy (phospholipon G), dimyristoylphosphatidylglycerole, lyso-phophatidylcholine, dipalmitoylphosphatidylglycerole, and/or their physiologically acceptable salts, desoxycholic acid, cholic acid, lithocholic acid, chendodesoxycholic acid, hyodesoxycholic acid, trihydroxycoprostanic acid, ursodesoxycholic acid, taurocholic acid, or glycocholic acid and/or their physiologically acceptable salts, as well as ethers of ethoxylated alcohols and alkyl-alcohols (C6-C16), alkyl-ester with C8-C20 with ethoxylated alcohols, ester of saturated and unsatured acids with C8-C20 with sugars, alkylethersulfates like polyether of caster oil and ethylene oxide (cremephor EL), polyoxyethylene fatty alcohol ether, polysorbic monoester, poloxamer, poloxamine.

According to another preferred embodiment of the present invention the system additionally comprises an alcohol.
Particularly preferred alcohols are C2-C8 alcohols, and in particular ethanol, propylene glycol, and glycerine.

In a preferred embodiment of the present invention the mass ratio of the lipophilic substance to the surfactant is preferably between 10 : 1 and 1 : 10 weight %, more preferably from 1 : 0.2 and 1; 1.5 weight %.

The concentration of the surfactant in the micro-emulsion system is preferably between 0.5 and 50 weight %, in particular between 5 and 25 weight %.

The pH value of the system according to the present invention is neutral and ranges preferably between 5.0 and 9.0, more preferably between 6.0 and 8.0.

Under the term adipose tissue disease and/or condition in particular any unwanted local fat deposits and/or the following disease examples including simple unaesthetic appearances like cellulite are understood:

Lipomae are benign slow growing tumors of fat cells, preferred located in the subcutaneous fatty tissue that can occur in various forms and characteristics. They can build mucus, chalk and/or become ossified. Additionally, increased built of connective tissue and capsules can occur together with newly built blood vessels which are all classified as abnormal because the compression on the blood vessels as well as on the nerve cells is algetic. Lipomae occur in various syndromes like for example the Gardner syndrome, the Lanois-Bensaude syndrome, and the Proteus syndrome.

Lipomatosis dolorosa and Cellulite are special forms of hypertrophic proliferation of fatty tissue which is located between the dermal fatty fascia and the underside of the dermis. Due to hormonal influences an enhanced capability to bind water in these fatty cells is observed which themselves initiate pressure and cause subsequently congestions in the lymphatic vessels. Additionally, compression and irritation to the peripheral sensitive nerves is applied so that the patients have an extreme sensitivity to contact. Over the years, irregular disseminated localised fatty nodes can built under the thinning dermis which are painful and show an unaesthetic character.

In this context also conditions like Lipoedema or lipodystrophic syndrome have to be mentioned.

The above addressed fatty tissue diseases demonstrate in contrast to alimentary related adipose disease pathophysiological tissue conditions that can be identified by histological scar and inflammation parameter as well as modifications in the histological fatty tissue morphology.

Under the term regression it is in particular understood that the lipolysis of the fatty tissue and the degeneration of the prolific fatty tissue is taking place.

The treatment with a formulation of the present invention is preferably directed to cellulite tissue and/or local deposits of unaestethic fatty tissue. In contrast to the known treatments especially the areas of mainly unaesthetic character are very receptive to the beneficial effect of a good biocompatibility and an enhanced bioavailability such that wider areas can be reached with a single application.

In general, all unwanted and/or unaesthetic fatty tissue can be treated with the formulations of the present invention. This includes adipose tissue around the eyes, at the cheeks, in the neck and chin region, at the back, under and around the arms, at the tighs, in the upper and lower stomach region, at the knee, and/or so called lovehandles by males, gluteal bananas by females, and saddlebacks.

In particular, with the use of micro-emulsions according to the present invention local deposits of unseasthetic fatty tissue around the eyes, under the arms, in the neck and chin region and/or at the tights are preferably treated. Those body regions often show a high sensitivity so that a possible reduction of injection points and injection frequency is most beneficial especially for the tissue in the mentioned regions.

The preparation of a micro-emulsion system of the present invention can for instance be such that at least one surfactant and at least one lipophilic substance are mixed in water in a ratio disclosed above. The preparation can be brought forward by any known form of preparation of micro-emulsions.

Application of a micro-emulsion system of the present invention can be carried out by any form of injection or topical application, in particular by subcutaneous injection, by intra-artery injection, by intra-muscular injection or by intravenous injection.
A percutaneous application is also possible in various carrier media. There, various aiding techniques like iontophoresis can be applied. The application can for instance be made via hydrostatic pressure. Thus, an even distribution is achieved.

Preferably, each unit of the formulation has a distinct dose of the micro-emulsion system as active ingredient. This dose can reach from about 10 mg to about 3000 mg, preferred from about 100 mg to about 1000 mg, per overall weight of the surfactant.

For the treatment of an adult patient by application of injection solutions day doses of 5 mg to 5000 mg, preferred of 250 mg to 2500 mg per injection per overall weight of the surfactant dependant of the size of the fatty tissue to be treated are administered.
The dose is also dependant to the size of the fat depot and/or the disordered distribution of the fat cells and/or the type of adipose disease. It should be tailored to the needs of the single patient. In the case of small lipomae even amounts of 10 mg to 50 mg can be used.

## Claims

1. Use of a micro-emulsion comprising at least one surfactant having a HLB value between 5 and 15, at least one lipophilic substance, and water for the preparation of a formulation for the treatment of adipose tissue disease and/or condition.

2. Use according to claim 1, **characterized in that** the micro-emulsion has a particle size between 5 nm and 200 nm, preferably between 30 nm and 100 nm.

3. Use according to claim 1 or 2, **characterized in that** the micro-emulsion additionally comprises an alcohol.

4. Use according to one or more of the preceding claims, **characterized in that** the at least one surfactant has an HLB value of between 6 and 12.

5. Use according to one or more of the preceding claims, **characterized in that** the at least one surfactant is selected from the group consisting of 3-sn-phosphatidylcholine, soy (phospholipone 90), reduced soy (phospholipon 9DH), 3-(3sn)-phosphatidylgycerol soy (phospholipon G), dimyristoylphosphatidylglycerole, lyso-phophatidylcholine, dipalmitoylphosphatidylglycerole, and/or their physiologically acceptable salts, desoxycholic acid, cholic acid, lithocholic acid, chendodesoxycholic acid, hyodesoxycholic acid, trihydroxycoprostanic acid, ursodesoxycholic acid, taurocholic acid, or glycocholic acid and/or their physiologically acceptable salts, as well as ethers of ethoxylated alcohols and alkyl-alcohols (C6-C16), alkyl-ester with C8-C20 with ethoxylated alcohols, ester of saturated and unsatured acids with C8-C20 with sugars, alkylethersulfates like polyether of caster oil and ethylene oxide (cremephor EL), polyoxyethylene fatty alcohol ether, polysorbic monoester, poloxamer, poloxamine.

6. Use according to one or more of the preceding claims, **characterized in that** the at least one lipophilic substance is selected from the group consisting of natural oils, ester of middle-chain alkylacids with glycols, octyl-dodecanol, silicon oils, paraffins.

7. Use according to one or more of the preceding claims, **characterized in that** the micro-emulsion additionally comprises at least one co-surfactant.

8. Use according to one or more of the preceding claims, **characterized in that** the treatment is directed to cellulite tissue and/or local deposits of unaestethic fatty tissue.

9. Use according to claim 8 **characterized in that** local deposits of unseasthetic fatty tissue around the eyes, under the arms, in the neck and chin region and/or at the tights are treated.
